# EUROPÄISCHE PATENTSCHRIFT

(11) **EP 0 548 302 B1**
(45) Veröffentlichungstag und Bekanntmachung des Hinweises auf die Patenterteilung: **04.09.1996**
(21) Anmeldenummer: 92912251.3
(22) Anmeldetag: 22.06.1992
(51) Int. Cl.: B25B 7/14, A61B 17/28

(54) **ARRETIERUNGSEINRICHTUNG AN EINEM ZANGENÄHNLICHEN WERKZEUG**
LOCKING SYSTEM FOR A FORCEPS LIKE INSTRUMENT
DISPOSITIF DE BLOCAGE POUR OUTILS SIMILAIRES A UNE PINCE

(30) Priorität: 09.07.1991 CH 2036/91
(43) Veröffentlichungstag der Anmeldung: 30.06.1993
(73) Patentinhaber: Synthes AG, Chur, CH-7002 Chur (CH)
(72) Erfinder: MATHYS, Robert, CH-2544 Bettlach (CH)
(74) Vertreter: Lusuardi, Werther Giovanni, Dr.
(86) Internationale Anmeldenummer: CH9200118
(87) Internationale Veröffentlichungsnummer: WO9301025

(56) Entgegenhaltungen:
- DE-C- 827 927
- US-A- 1 944 116
- US-A- 2 881 649
- US-A- 4 538 485

## Beschreibung

Die Erfindung betrifft eine Arretierungseinrichtung an einem zangenartigen Werkzeug nach dem Oberbegriff des Patentanspruchs 1. Die Arretierungseinrichtung bewirkt, dass das zangenartige Werkzeug ein Objekt festklemmen, festgeklemmt halten und wieder loslassen kann.

Ein zangenartiges Werkzeug besteht aus zwei Schenkeln, die um einen Drehpunkt gegeneinander beweglich sind. Auf der einen Seite des Drehpunktes bilden die Schenkel mit je einer Lippe an jedem Schenkel ein Maul, auf der anderen Seite ist jeder Schenkel als Griff ausgebildet und trägt dort eventuell Fingerlöcher. Die Erfindung wird an Hand von Zangen als häufigstes zangenartiges Werkzeug erklärt.

Arretierungseinrichtungen für zangenartige Werkzeuge sind bereits bekannt.

So existieren Zangen, bei denen sich am einen Schenkel eine beweglich angebrachte lange Schraube befindet, die durch ein Loch im zweiten Schenkel geführt ist. Sie trägt vor der Aussenseite des zweiten Schenkels eine Mutter. Diese wird bis zur Aussenseite des zweiten Schenkels geschraubt, nachdem man mit dem Maul der Zange das Objekt ergriffen hat, so dass das Objekt in das Maul der Zange festgeklemmt wird. Das Objekt wird wieder frei, wenn man die Mutter genügend weit löst. Diese Vorrichtung ist auch bei sehr steifer Konstruktion der Zange wegen der kontinuierlichen Einstellbarkeit der Mutter für jeden beliebigen Durchmesser des Objektes innerhalb der Maulweite der Zange verwendbar, sie benötigt aber zur Bedienung zwei Hände. Die Schraube steht über das Volumen der Stange hinaus und kann so die Anwendung der Zange bei beschränkten Platzverhältnissen, wie sie beispielsweise in der Chirurgie vorherrschen, beeinträchtigen.

Bei einer weiteren Zange sind an den maulfernen Enden der Innenseite der Schenkel aufeinander zulaufend je ein streifenförmiges Stück angefügt. Diese streifenförmigen Stücke laufen eng aneinander vorbei. An den einander zugewandten Seiten tragen sie mehrfache, dreieckförmige Ausformungen, so dass die Schenkel durch Verhaken der Dreiecke auf den Streifen in verschiedenen Abständen voneinander feststellbar sind. Bei einer elastisch-weichen Konstruktion der Zange, beispielsweise bei langen Schenkeln, lässt sich jedes Objekt innerhalb der maximalen Maulweite der Zange festklemmen, wobei jedoch der Festklemmdruck je nach Position der Schenkel verschieden ist. Das einhändige Freilassen des Objektes ist nur möglich, wenn die Schenkel wie Scheren am Ende Fingerlöcher tragen, auch dann erfordert beim Freilassen des Objektes aus der Zange das Lösen der Verspannung der Schenkel quer zur Öffnungsrichtung einen gewissen unphysiologischen Kraftaufwand.

Eine weitere Arretierungseinrichtung für eine Zange ist in der WO89/06939 beschrieben. Bei dieser Zange trägt ein erster Schenkel auf seiner Innenseite zwischen dem Drehpunkt und dem Griff eine Zahnstange, die durch eine Öffnung im zweiten Schenkel hindurchgeht. An der Öffnung des zweiten Schenkels befindet sich eine Sperrklinke, die in drei verschiedenen Stellungen so auf die Zahnstange einwirkt, dass bei der ersten Stellung die Zange nur geschlossen werden kann, in der zweiten Stellung die Zange nur geöffnet werden kann und in der dritten Stellung die Zange ungehindert geschlossen oder geöffnet werden kann. In der ersten Stellung klemmt die Zange also ein Objekt fest, sobald sich das Maul auf dessen Durchmesser eingestellt hat. Sie lässt es wieder los, wenn eine andere Stellung eingenommen ist. Die Stellungen werden an einer mit der Sperrklinke koaxialen Umschalteinrichtung eingestellt und durch eine an einer Druckfeder befindlichen Kugel, die in die Sperrklinke jeweils in einer der drei Stellungen eingreift, aufrecht erhalten. Diese Zange lässt sich einhändig bedienen. Sie benötigt aber, da die Zahnstange durch den zweiten Schenkel in den Aussenraum der Zange ragt, ähnlich viel Platz wie die erstgenannte Zange mit der Schraube.

Aus der US 4 538 485 SAILA ist schliesslich eine gattungsgemässe Zange bekannt, bei welcher der Hebel der Arretierungseinrichtung nach hinten über die beiden Schenkel hinausragt. Der nach hinten hinausragende Hebel kann zu Verletzungen führen und muss, um eine unbeabsichtigte Betätigung der Arretierungseinrichtung zu verhindern, mittels eines zusätzlichen Mechanismus gesichert werden. Besonders nachteilig ist bei dieser bekannten Vorrichtung jedoch der Umstand, dass der nach hinten hinausragende Hebel nicht mit der gleichen, die Zange haltenden Hand, betätigt werden kann und der Operateur gezwungen ist, beide Hände zur Betätigung der Zange zu benutzen.

Der Erfindung liegt die Aufgabe zugrunde, eine Arretierungseinrichtung an einem zangenartigen Werkzeug zu schaffen, welche vollständig im Inneren der Zange liegt, aber trotzdem einfach und sicher mit einer einzigen Hand bedienbar ist.

Diese Aufgabe wird durch die im Kennzeichenteil des Patentanspruchs 1 angeführten Merkmale gelöst. Die weiteren Ansprüche betreffen vorteilhafte Ausführungsformen.

Die Erfindung wird beispielhaft an Hand der Zeichnung erklärt. Dabei zeigen
- Fig. 1a: eine Zange mit einer Arretierungseinrichtung in einer Offenstellung mit herausgezeichneter Hebelpartie,
- Fig. 1b: dieselbe Zange in Schliessstellung,
- Fig. 2: eine Rastervorrichtung in der Form einer Zahnstange bei rechteckigem Querschnitt einer Formstange,
- Fig. 3: eine Rastervorrichtung in der Form von Eindrehungen bei kreisförmigem Querschnitt einer Formstange,
- Fig. 4a: eine Zange mit einer anderen Arretierungseinrichtung in Schliessstellung,
- Fig. 4b: einen Schnitt durch die Partie um den Hebel von Fig. 4a in Offenstellung,
- Fig. 4c: einen Schnitt wie in Fig. 4b in Schliessstellung.

In der Zeichnung sind in den verschiedenen Figuren die sich entsprechenden Bauteile mit den gleichen Bezugszeichen versehen.

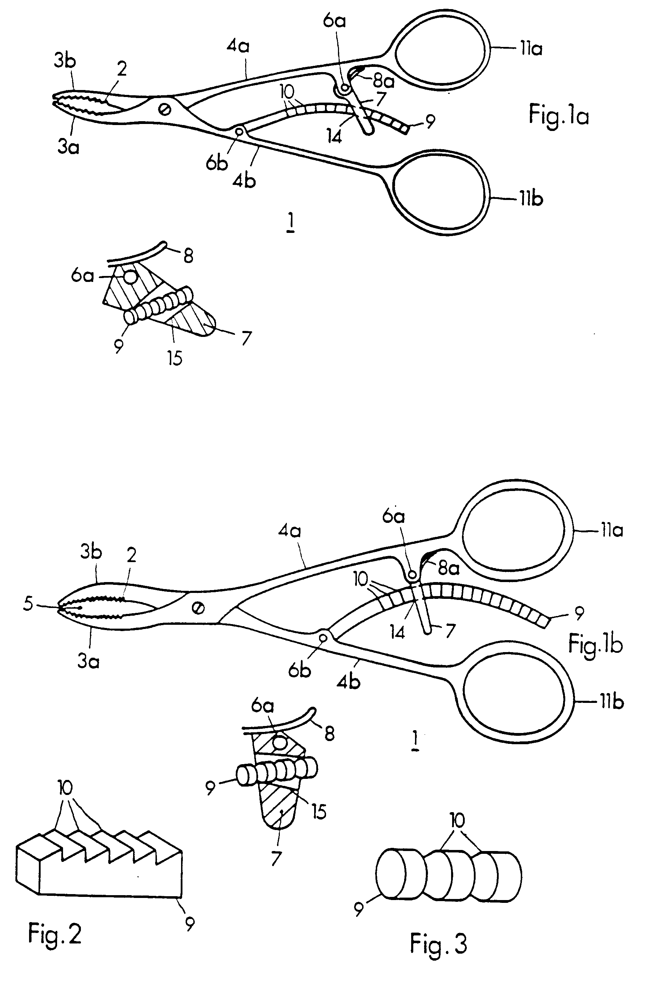

Die Figur 1a zeigt eine erfindungsgemässe Zange 1 in einer Stellung (Offenstellung), in der ihre Schenkel 4a, 4b ungehindert um den Drehpunkt beweglich sind, die Fig. 1b diese Zange in einer Stellung (Schliessstellung), in der ihre Schenkel 4a,4b nur geschlossen werden können.

Man erkennt an der Zange 1 ein Maul 2, dessen erste Lippe 3a und zweite Lippe 3b hier Zähne tragen, einen ersten Schenkel 4a und einen zweiten Schenkel 4b, die beide an der Griffseite in je einem Fingerloch 11a,11b enden. Um die Zange 1 zu bedienen, muss man den Daumen und den Mittelfinger je durch eines der Fingerlöcher 11a,11b stecken und mit dem Zeigefinger einen später beschriebenen Hebel 7 betätigen. In der Fig. 1b, welche die Zange 1 in der Schliessstellung zeigt, befindet sich in ihrem Maul 2 ein Objekt 5.

An dieser Zange 1 ist an der Innenseite des zweiten Schenkels 4b mittels eines Stiftes 6b eine Formstange 9 schwenkbar angeordnet. Sie trägt eine Rastervorrichtung 10, d.h. eine Anordnung von periodisch angeordneten Formelementen, die weiter unten in Beispielen beschrieben wird. Ferner besitzt sie einen an der Innenseite des ersten Schenkels 4a durch einen Stift 6a angeordneten Hebel 7. Dieser Hebel 7 ist in beiden Figuren 1a, 1b gesondert herausgezeichnet.

Der Hebel 7 und die Formstange 9 liegen vollständig zwischen den Schenkeln 4a,4b und ragen an keiner Stelle über diese hinaus. Der Hebel 7 besitzt eine Öffnung 14, durch welche die Formstange 9 läuft. Er ist so gestaltet, dass er nur zwei stabile Stellungen, eine Offenstellung und eine Schliessstellung, einnehmen kann. Dies wird in dem gegebenen Beispiel dadurch erreicht, dass er an seinem dem Schenkel 4a zugewandten Ende winkelig ausgebildet ist und an einem ebenen Stück einer Blattfeder 8a anliegt. Er kann, schwenkbar um den Stift 6a, an der einen Winkelseite oder an der anderen Winkelseite an der Feder 8a anliegen. In jeder Zwischenstellung wird er durch die Feder 8a in die Offenstellung oder in die Schliessstellung zurückgeführt.

An seiner Öffnung 14 besitzt der Hebel 7 einen scharfen Vorsprung 15, mit dem er in Schliessstellung in die Rastervorrichtung 10 der Formstange 9 eingreift. Dieser Eingriff erfolgt in einer Weise, dass die Zange 1 nur noch schliessbar ist. Um dies zu erreichen, ist die Formstange 9 so gestaltet, dass der Hebel 7 und die griffseitige Kante seines Vorsprungs 15 in der Schliessstellung stets senkrecht oder nahezu senkrecht auf der Formstange 9 steht.
Die Formelemente der Rastervorrichtung 10 der Formstange 9 besitzen jeweils gegen die Griffseite der Schenkel 4a,4b hin gerichtet, eine ebenfalls senkrecht zu der Richtung der Formstange 9 verlaufende Kante, hinter die der scharfe Vorsprung 15 des Hebels 7 eingreift, wodurch der Hebel 7 sich nicht mehr nach der Griffseite der Schenkel 4a,4b verschieben lässt. Die Schenkel 4a,4b und damit die Zange 1 lassen sich dann nicht mehr öffnen. Befindet sich ein Objekt 5 im Maul der Zange 1, so wird es festgeklemmt, sobald das Maul 2 sich um das Objekt geschlossen hat und wird dann automatisch von der Zange 1 festgeklemmt gehalten.

In der Offenstellung liegt die Formstange 9 auf einer breiten Fläche auf. Sie kann dann ungehindert durch die Öffnung 14 laufen und die Zange 1 lässt sich ungehindert öffnen und schliessen. Ein im Maul 2 der Zange 1 festgeklemmtes Objekt 5 wird freigegeben, sobald der Hebel 7 mit einem Finger der Betätigungshand in die Offenstellung gebracht ist.

Beispiele für die Ausbildung der Rastervorrichtung 10 an der Formstange 9 zeigen die Fig. 2, wobei eine rechteckige Formstange 9 als Zahnstange ausgebildet ist, und die Fig. 3, wobei eine Formstange 9 mit kreisförmigen Querschnitt Eindrehungen in der Form von Ringnuten trägt. Die Formstange kann aber auch mit einem Schraubengewinde oder in einer anderen geeigneten Form ausgebildet sein. In allen Fällen sind die Formelemente der Rastervorrichtung 10 asymmetrisch ausgebildet mit einer in Richtung zu den Griffen liegenden senkrecht zur Richtung der Formstange 9 verlaufenden Kante.

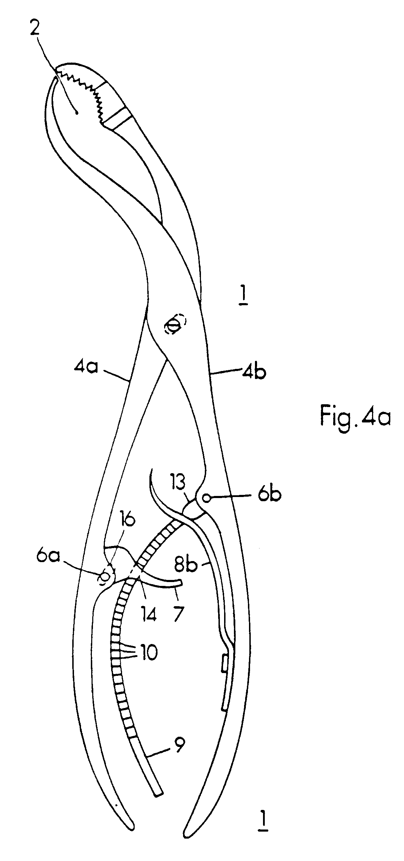

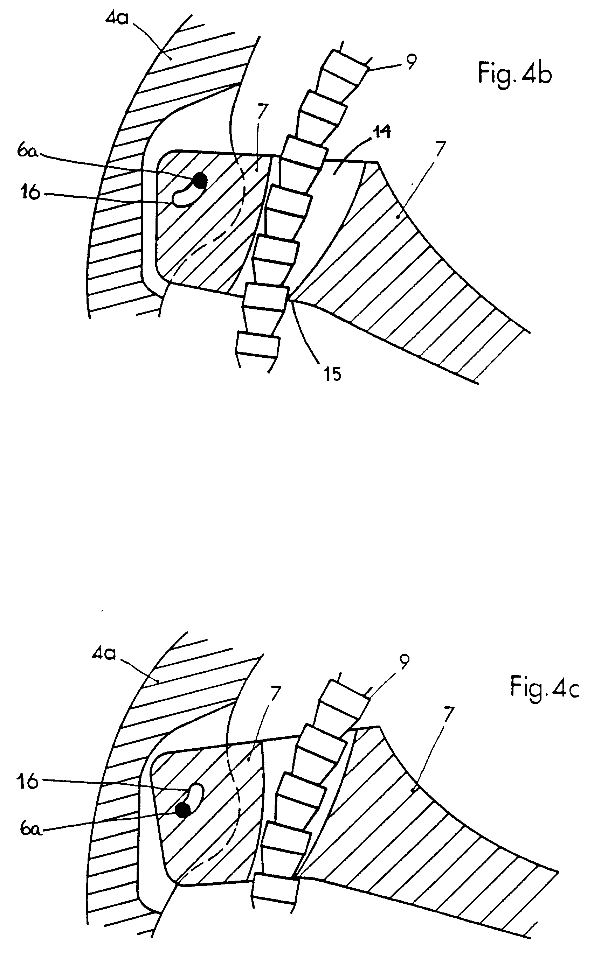

Die Fig. 4a zeigt eine andere Ausführung der erfindungsgemässen Zange 1 in der Schliessstellung, die Fig. 4b die Partie um ihren Hebel 7 in der Offenstellung, die Fig. 4c die Partie um ihren Hebel in der Schliessstellung. Alle drei Figuren werden gemeinsam besprochen.

In der Offenstellung des Hebels 7 läuft die Formstange 9 ungehindert in der Öffnung 14 des Hebels 7, die Zange 1 kann geöffnet und geschlossen werden. Die Formstange 9 ist um den Stift 6b schwenkbar mit dem zweiten Schenkel 4b der Zange 1 verbunden. Durch eine geeignet - hier S-förmig - geformte Feder 8b am zweiten Schenkel 4b drückt, geführt vom Gleitstein 13, die Formstange 9 über den Hebel 7 gegen den ersten Schenkel 4a, so dass die Zange 1 den maximal geöffneten Zustand anstrebt und nur schliessbar ist, indem man die beiden Schenkel 4a, 4b gegeneinander drückt.

Durch diesen Zwang zum Öffnen der Zange 1 kann die Arretierungseinrichtung anders ausgeführt werden als oben beschrieben. Der Hebel 7 besitzt ein halbmondförmig ausgebildetes Schlitzloch 16, in dem der Stift 6a läuft, der den Hebel 7 mit dem ersten Schenkel 4a verbindet. Auch hier ist der Hebel 7 zweier stabiler Stellungen fähig. In der Offenstellung befindet sich der Stift 6a in der maulseitigen Hälfte des Schlitzloches 16, wie in der Fig. 4b gezeichnet. Durch die Spannung, welche die Feder 8a auf die Formstange 9 ausübt, wird diese auf der dem ersten Schenkel 4a zugewandten Seite der Öffnung 14 im Hebel 7 geführt und läuft ohne Behinderung auf einer breiten Fläche der Öffnung 14.

Legt man den Hebel 7 in die Schliessstellung um, so geht der Stift 6a in die griffseitige Hälfte des Schlitzloches 16 über und der Hebel 7 steht nahezu senkrecht auf der Formstange 9. Der scharfe Vorsprung 15 in der Öffnung 14 des Hebels 7 kann nun in die Rastervorrichtung 10 der Formstange 9 eingreifen und lässt nur noch ein Schliessen der Zange 1 zu. Der Hebel 7 ist somit nur zweier stabiler Stellungen fähig, der Schliessstellung und der Offenstellung. Die übrigen Teile der Zange 1 haben dieselben Bezeichnungen und üben dieselben Funktionen aus wie in den Figuren 1a, 1b.

Die Zange 1 klemmt somit ein Objekt 5 (nicht gezeichnet) automatisch in ihrem Maul 2 fest, wenn sie in der Schliessstellung so weit geschlossen ist, dass das Objekt 5 vom Maul der Zange erfasst wird. Sie kann es erst wieder loslassen, wenn der Hebel 7 mit einem Finger der Betätigungshand in die Offenstellung umgelegt ist.

Bei den erfindungsgemässen Arretierungseinrichtungen, von denen Beispiele in den Fig. 1a, 1b und 4a dargestellt sind, besitzt der Hebel 7 eine Öffnung 14, durch die eine Formstange 9 läuft, die eine Rastervorrichtung 10 trägt. Der Hebel 7 und die Formstange 9 sind vollständig zwischen den Schenkeln 4a, 4b der Zange angeordnet. Der Hebel 7 ist so gestaltet, dass er durch Federdruck nur zwei stabile Stellungen, eine Offenstellung und eine Schliessstellung, einnehmen kann. Er weist innerhalb der Öffnung 14 einen scharfen Vorsprung 15 auf, mit dem er in der Schliessstellung in die Rastervorrichtung 10 der Formstange 9 eingreift, so dass die Zange 1 nur noch schliessbar ist. In der Offenstellung läuft die Formstange 9 dagegen über breite Flächen der Öffnung 14 am Hebel 7, so dass die Zange 1 ungehindert geöffnet und geschlossen werden kann.

Die erfindungsgemässe Arretierungseinrichtung lässt sich auch bei Zangen 1 verwenden, bei denen man, beispielsweise durch ein Schlitzloch am Drehpunkt zwischen den beiden Schenkeln 4a, 4b, die Maulweite und die Gestalt des Mauls 2 variieren kann (sogenannte selbstzentrierende Zangen).

Die erfindungsgemässe Arretierungseinrichtung erfüllt die Aufgabestellung, da sie eine einhändige Bedienung von zangenartigen Werkzeugen ermöglicht und da sie völlig innerhalb der Schenkel 4a, 4b des zangenartigen Werkzeuges liegt. Die mit ihr ausgerüsteten zangenartigen Werkzeuge können allgemein in der Industrie, im Handwerk und von Heimwerkern angewendet werden, sie finden aber vor allem Anwendung in der Chirurgie.

## Patentansprüche

1. Arretierungseinrichtung an einem zangenartigen Werkzeug, vornehmlich an einer Zange (1), die an einem ersten Schenkel (4a) einen in verschiedene Stellungen umschaltbaren Klemmechanismus und an der Innenseite eines zweiten Schenkels (4b) eine Formstange (9) mit einer Rastervorrichtung (10) aufweist, wobei der Klemmechanismus in die Formstange (9) eingreift,
dadurch gekennzeichnet, dass
- der Klemmechanismus einen einteiligen Hebel (7) umfasst,
- der Hebel (7) und die Formstange (9) in jeder Stellung der beiden Schenkel (4a, 4b) vollständig zwischen diesen beiden Schenkeln (4a, 4b) angeordnet sind und an keiner Stelle über diese hinausragen,
- der Hebel (7) eine Öffnung (14) besitzt, durch welche die Formstange (9) läuft,
- der Hebel (7) so gestaltet ist, dass er nur zwei stabile Stellungen, eine Offenstellung und eine Schliessstellung, einnehmen kann,
- der Hebel (7) innerhalb der Öffnung (14) einen scharfen Vorsprung (15) aufweist, mit dem er in der Schliessstellung so in die Rastervorrichtung (10) der Formstange (9) eingreift, dass die Zange (1) nur schliessbar ist,
- der Hebel (7) innerhalb der Öffnung (14) Flächen aufweist, zwischen denen in der Offenstellung die Formstange (9) ungehindert bewegbar ist, so dass die Zange (1) sowohl aufmachbar als auch schliessbar ist; und
- der Hebel (7) einhändig bedienbar ist.

2. Arretierungseinrichtung nach Anspruch 1, dadurch gekennzeichnet, dass der Hebel (7) an seinem dem ersten Schenkel (4a) zugewandten Ende winkelig ausgebildet ist und in der Offenstellung die eine Winkelfläche, in der Schliessstellung die andere Winkelfläche an einem ebenen Stück einer ersten Feder (8a) anliegt, bei Zwischenstellungen der Hebel (7) durch die Feder (8a) in die Offenstellung oder in die Schliessstellung zurückgeführt wird.

3. Arretierungseinrichtung nach Anspruch 1, dadurch gekennzeichnet, dass am zweiten Schenkel (4b) eine zweite Feder (8b) angebracht ist, die so geformt ist, dass die Zange (1) stets den maximal geöffneten Zustand anstrebt.

4. Arretierungseinrichtung nach Anspruch 3, dadurch gekennzeichnet, dass die zweite Feder (8b) S-förmig gebogen ist und eine Öffnung für die Formstange (9) besitzt.

5. Arretierungseinrichtung nach Anspruch 3 oder 4, dadurch gekennzeichnet, dass die zweite Feder (8b) so geformt ist, dass sie die an einem zweiten Stift (6b) schwenkbar am zweiten Schenkel (4b) befestigte Formstange (9) über den Hebel (7) gegen den ersten Schenkel (4a) drückt.

6. Arretierungseinrichtung nach einem der Ansprüche 3 bis 5, dadurch gekennzeichnet, dass die zweite Feder (8b) mittels eines Gleitsteines (13) auf die Formstange (9) wirkt.

7. Arretierungseinrichtung nach Anspruch 3, dadurch gekennzeichnet, dass der Hebel (7) ein halbmondförmiges Schlitzloch (16) besitzt, in dem ein erster Stift (6a) die Verbindung zu dem ersten Schenkel (4a) bildet und sich dieser erste Stift (6a) in der Offenstellung des Hebels (7) in der maulseitigen Hälfte des halbmondförmigen Schlitzloches (16), in der Schliessstellung in der griffseitigen Hälfte des halbmondförmigen Schlitzloches (16) befindet.

8. Arretierungseinrichtung nach Anspruch 3, dadurch gekennzeichnet, dass der erste Schenkel (4a) ein halbmondförmiges Schlitzloch (16) besitzt, in dem ein erster Stift (6a) die Verbindung zum Hebel (7) bildet und dieser erste Stift (6a) sich bei der Offenstellung des Hebels (7) in der maulseitigen Hälfte des halbmondförmigen Schlitzloches (16), in der Schliessstellung in der griffseitigen Hälfte des halbmondförmigen Schlitzloches (16) befindet.

9. Arretierungseinrichtung nach Anspruch 1, dadurch gekennzeichnet, dass in der Schliessstellung der Hebel (7) senkrecht auf der Formstange (9) steht.

10. Arretierungseinrichtung nach Anspruch 1, dadurch gekennzeichnet, dass die Formstange (9) einen kreisförmigen Querschnitt besitzt und die Rastervorrichtung (10) die Form eines Schraubengewindes oder von periodisch angebrachten Ringnuten besitzt.

11. Arretierungseinrichtung nach Anspruch 1, dadurch gekennzeichnet dass die Formstange (9) einen Querschnitt mit mindestens zwei Kanten besitzt und die Rastervorrichtung (10) als Zahnstange ausgebildet ist.

12. Arretierungseinrichtung nach einem der Ansprüche 9 bis 11, dadurch gekennzeichnet dass die Rastervorrichtung (10) an der Formstange (9) in Griffrichtung senkrecht zur Formstange verlaufende Kanten aufweist.

13. Arretierungseinrichtung nach Anspruch 1, dadurch gekennzeichnet, dass die Rastervorrichtung (10) der Formstange (9) eine Periode besitzt, die kleiner ist als die Zusammendrückbarkeit der Schenkel (4a, 4b) der Zange (1) beim Schliessen.

## Claims

1. A locking device at a pliers-shaped tool, preferably in a pair of pliers (1) which comprises at a first handle (4a) a gripping mechanism that can be moved to different settings and at the inner side of a second handle (4b) a shaped rod (9) with a catching device (10), by means of which the gripping mechanism hooks into the shaped rod (9),
**characterized in that**
- the gripping mechanism comprises a one-piece lever (7);
- the lever (7) and the shaped rod (9) in every position of the two handles (4a, 4b) are entirely between these two handles (4a, 4b) and do not project over the latter at any point;
- the lever (7) has an opening (14) through which the shaped rod (9) moves;
- the lever (7) is configured in such a way that it can assume only two stable settings, an open setting and a closed setting;
- the lever (7) has a sharp projection (15) within the opening (14), with which it hooks onto the catching device (10) of the shaped rod (9) when in the closed setting, so that the pliers (1) can only be closed;
- the lever (7) within the opening (14) has surfaces between which, in the open position, the shaped rod (9) is freely movable, so that the pliers (1) can be opened as well as closed; and
- the lever (7) is operable with a single hand.

2. The locking device according to claim 1, characterized in that the end of the lever (7) which faces the first handle (4a) is angle-configured and in the open setting one angle surface adjoins a flat piece of the first spring (8a), and in the closed setting the other angle surface adjoins it, in intermediate settings the lever (7) is brought back either to the open setting or the closed setting by the spring (8a).

3. The locking device according to claim 1, characterized in that on the second handle (4b) a second spring (8b) is located which is so configured that the pliers (1) constantly tends toward the most open position possible.

4. The locking device according to claim 3, characterized in that the second spring (8b) is bent in a S-shape and has an opening for the shaped rod (9).

5. The locking device according to claim 3 or 4, characterized in that the second spring (8b) is configured in such a way that it presses the shaped rod (9), which is swingingly attached by a second pin (6b) to the second handle (4b), against the first handle (4a) by means of the lever (7).

6. The locking device according to one of the claims 3 to 5, characterized in that the second spring (8b) acts on the shaped rod (9) by means of a sliding pad (13).

7. The locking device according to claim 3, characterized in that the lever (7) has a crescent-shaped slot hole (16), in which a first pin (6a) forms the link to the first handle (4a) and in the open setting of the lever (7), this first pin (6a) is in the section of the crescent-shaped slot hole (16) that is closer to the jaws, while in the closed setting it is in the section of the crescent-shaped slot hole (16) that is closer to the grip ends.

8. The locking device according to claim 3, characterized in that the first handle (4a) has a crescent-shaped slot hole (16), in which the first pin (6a) forms the link to the lever (7), and in the open setting of the lever (7) this first pin (6a) is in the section of the crescent-shaped slot hole (16) that is closer to the jaws, while in the closed setting it is in the section of the crescent-shaped slot hole (16) that is closer to the grip end.

9. The locking device according to claim 1, characterized in that in the closed setting, the lever (7) stands perpendicular to the shaped rod (9).

10. The locking device according to claim 1, characterized in that the shaped rod (9) has a circular cross section, and the catching device (10) has the form of a screw thread or of periodically placed ring grooves.

11. The locking device according to claim 1, characterized in that the shaped rod (9) has a cross section with at least two edges, and the catching device (10) is configured as a toothed rack.

12. The locking device according to one of the claims 9 to 11, characterized in that the catching device (10) on the shaped rod (9) has edges running perpendicular to the shaped rod (9) in the direction of the grip end.

13. The locking device according to claim 1, characterized in that the catching device (10) of the shaped rod (9) has a periodicity that is smaller than the compressibility of the handles (4a and 4b) of the pliers (1) during closing.

## Revendications

1. Dispositif de blocage sur un outil du type pince, principalement sur une pince (1) qui présente sur un premier bras (4a) un mécanisme de pinçage pouvant être basculé en différentes positions, et sur le côté interne d'un second bras (4b) une tige façonnée (9) comportant un dispositif d'encliquetage (10), le mécanisme de pinçage s'engageant dans la tige façonnée (9), caractérisé en ce que:
- le mécanisme de pinçage comprend un levier (7) d'une seule pièce,
- dans chaque position des deux bras (4a, 4b) le levier (7) et la tige façonnée (9) sont disposés entièrement entre ces deux bras (4a, 4b), et ne débordent au-delà de ceux-ci en aucun endroit,
- le levier (7) possède une ouverture (14) traversée par la tige façonnée (9),
- le levier (7) est configuré de manière à ne pouvoir prendre que deux positions stables, une position d'ouverture et une position de fermeture,
- le levier (7) présente une saillie aiguë (15) à l'intérieur de l'ouverture (14), par laquelle il s'engage en position de fermeture dans le dispositif d'encliquetage (10) de la tige façonnée (9), de telle sorte que la pince (1) puisse uniquement être fermée,
- le levier (7) présente à l'intérieur de l'ouverture (14) des surfaces entre lesquelles la tige façonnée (9) peut être déplacée sans obstacle dans la position d'ouverture, de sorte que la pince (1) peut être aussi bien ouverte que fermée; et
- le levier (7) peut être actionné à une seule main.

2. Dispositif de blocage selon la revendication 1, caractérisé en ce que le levier (7) est configuré en forme d'angle à son extrémité tournée vers le premier bras (4a) et, dans la position d'ouverture, une des surfaces de l'angle se place contre une pièce plane d'un premier ressort (8a), l'autre surface de l'angle étant placée sur cette pièce plane dans la position de fermeture, tandis que dans les positions intermédiaires le levier (7) est ramené par le ressort (8a) dans la position d'ouverture ou dans la position de fermeture.

3. Dispositif de blocage selon la revendication 1, caractérisé en ce que sur le second bras (4b) est installé un second ressort (8b) qui présente une forme telle que la pince (1) tend toujours à prendre sa position d'ouverture maximale.

4. Dispositif de blocage selon la revendication 3, caractérisé en ce que le second ressort (8b) est incurvé en forme de S et possède une ouverture pour la tige façonnée (9).

5. Dispositif de blocage selon la revendication 3 ou 4, caractérisé en ce que le second ressort (8b) est configuré de manière à repousser contre le premier bras (4a) par l'intermédiaire du levier (7), la tige façonnée (9) fixée à pivotement sur un second pivot (6b) du second bras (4b).

6. Dispositif de blocage selon l'une quelconque des revendications 3 à 5, caractérisé en ce que le second ressort (8b) agit sur la tige façonnée (9) au moyen d'une tête de coulissement (13).

7. Dispositif de blocage selon la revendication 3, caractérisé en ce que le levier (7) possède un trou oblong (16) en forme de demi-lune dans lequel un premier pivot (6a) constitue la liaison avec le premier bras (4a), et dans la position d'ouverture du levier (7), ce premier pivot (6a) est situé dans la moitié du trou oblong (16) en forme de demi-lune qui est située du côté des mâchoires, tandis que dans la position de fermeture, il se trouve dans la moitié du trou oblong (16) en forme de demi-lune qui se trouve du côté des poignées.

8. Dispositif de blocage selon la revendication 3, caractérisé en ce que le premier bras (4a) possède un trou oblong (16) en forme de demi-lune, dans lequel un premier pivot (6a) constitue la liaison avec le levier (7), et dans la position d'ouverture du levier (7), ce premier pivot (6a) est situé dans la moitié du trou oblong (16) en forme de demi-lune qui est située du côté des mâchoires, tandis que dans la position de fermeture, il se trouve dans la moitié du trou oblong (16) en forme de demi-lune qui est située du côté des poignées.

9. Dispositif de blocage selon la revendication 1, caractérisé en ce que dans la position de fermeture, le levier (7) forme un angle droit avec la tige façonnée (9).

10. Dispositif de blocage selon la revendication 1, caractérisé en ce que la tige façonnée (9) possède une section transversale circulaire, et le dispositif d'encliquetage (10) la forme d'un filet hélicoïdal ou de rainures annulaires pratiquées à intervalles périodiques.

11. Dispositif de blocage selon la revendication 1, caractérisé en ce que la tige façonnée (9) possède une section transversale présentant au moins deux bords, et le dispositif d'encliquetage (10) est configuré comme tige dentée.

12. Dispositif de blocage selon l'une quelconque des revendications 9 à 11, caractérisé en ce que, dans la direction des poignées le dispositif d'encliquetage (10) de la tige façonnée (9) présente des bords s'étendant perpendiculairement à la tige façonnée.

13. Dispositif de blocage selon la revendication 1, caractérisé en ce que le dispositif d'encliquetage (10) de la tige façonnée (9) possède une période qui est inférieure à la possibilité de rapprochement des bras (4a, 4b) de la pince (1) lors de la fermeture.
